# EUROPEAN PATENT APPLICATION

(11) **EP 4 410 932 A1**
(43) Date of publication of application: **07.08.2024**
(21) Application number: 22875128.5
(22) Date of filing: 30.09.2022
(51) Int. Cl.: C10M 129/66, C10L 1/00, C10L 1/10, C10L 1/14, C10L 1/18, C10L 1/19, C10L 10/08, C10N 30/00, C10N 30/06, C10M 105/36, C10M 105/32, C10M 129/18, C10M 169/04, C07D 301/12, C07D 303/48, C10N 40/25

(54) **LUBRICITY IMPROVER AND APPLICATION THEREOF IN OIL PRODUCT**

(30) Priority: 30.09.2021 CN 202111155603
(71) Applicant: China Petroleum & Chemical Corporation, Beijing 100728 (CN); Sinopec Research Institute of Petroleum Processing Co., Ltd., Beijing 100083 (CN)
(72) Inventor: XIA, Xin, Beijing 100083 (CN); LIN, Jianmin, Beijing 100083 (CN); TAO, Zhiping, Beijing 100083 (CN); LI, Yan, Beijing 100083 (CN); LI, Baoshi, Beijing 100083 (CN)
(74) Representative: Hoffmann Eitle
(86) International application number: PCT/CN2022/122983
(87) International publication number: WO 2023/051747

(57) **Abstract**

A lubricity modifier and application thereof, the lubricity modifier comprising a compound having a structure represented by formula (I), wherein, groups R₁ and R₂ are each independently a single bond or a substituted or unsubstituted C₁-C₂₀ hydrocarbylene group, groups R₃ and R₄ are each independently hydrogen or a substituted or unsubstituted C₁-C₁₀ hydrocarbyl group, groups X and Y are each independently hydrogen or a substituted or unsubstituted C₁-C₅₀ hydrocarbyl group, and at least one of X and Y is hydrogen. The lubricity modifier product can significantly improve the lubricity of oil products at a low dosage in the oil products, has good stability, is not easy to oxidize and/or deteriorate, and can maintain good lubricity improving effect for a long time.

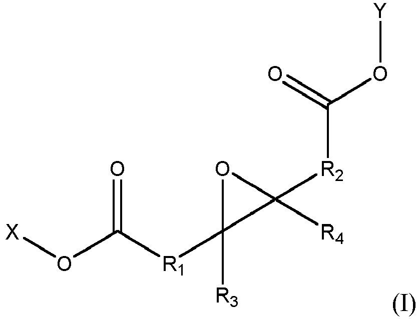

## Description

### Technical Field

The present application relates to the field of additives for oil products, in particular to an additive for improving lubricity of oil products and an oil product composition containing the additive.

### Background Art

In order to meet the environmental requirements, low-sulfur or ultra-low-sulfur diesel oil has become a target sought by various countries in the world. With the continuous improvement of the hydrofining process technology, the production of low-sulfur diesel oil is also accompanied by the removal of other components with lubricity, such as oxygen-containing and/or nitrogen-containing compounds and polycyclic aromatic hydrocarbon compounds. The deterioration in lubricity of the diesel oil will lead to abrasion during operation of a diesel engine, and thus it is difficult to ensure the normal operation of the engine. In order to improve lubricity of diesel oil, the addition of lubricity modifiers (also known as "antiwear additives") with the lubricating performance to low-sulfur diesel oil is currently the simplest, effective and economical method.

Chinese patent application CN109576063A discloses an antiwear additive for low-sulfur diesel oil and a preparation process thereof, wherein the process comprises mixing an esterification product of maleic anhydride, vegetable oil fatty acid with non-conjugated double bonds and a catalyst in proportion, subjecting the mixture to reaction for a certain time under stirring at 180-220°C, lowering the temperature after the reaction is completed, recovering the catalyst, and obtaining the antiwear additive product after vacuum distillation. Although the antiwear additive product prepared by this process shows good lubricity improving performance, the source of raw materials is relatively limited, which leads to a significant increase in the production cost of the antiwear additive product, not facilitating the industrial production. On the other hand, the prepared antiwear additive product has poor oxidation stability, thus it is difficult to maintain a stable lubricity improving effect, is not beneficial for the storage of the antiwear additive product, and has higher requirements on the storage of the product.

Chinese patent CN103540369B discloses an antiwear additive composition for low-sulfur diesel oil, which is composed of the following components in parts by mass: 30-50 parts of glycerol stearate, 10-20 parts of isocetyl palmitate, 10-20 parts of cetyl ethylhexanoate and 10-20 parts of Laneth-25. Although the antiwear additive product disclosed in this patent can improve the lubricity of low-sulfur diesel oil, the source of raw materials is limited, resulting in high preparation cost for the antiwear additive product.

Chinese patent CN101768480B discloses an antiwear additive for diesel oil, which is composed of raw materials (A) at least one amide component, (B) at least one amine oiliness additive component, (C) at least one ester oiliness additive component, (D) at least one rust inhibitor, (E) at least one demulsifier, and (F) at least one organic solvent. Although the antiwear additive product can improve the lubricity of low-sulfur diesel oil, the antiwear additive product contains nitrogen-containing component substances such as amine and amide compounds, and adding the antiwear additive product into diesel oil leads to the generation of nitrogen oxides, which is not in line with the concept of green chemistry.

Chinese patent CN109929624B discloses a fatty acid antiwear additive for diesel oil and a formulation thereof, which is specifically composed of 80 parts by weight of a fatty acid antiwear additive for diesel oil and 20 parts by weight of an organic nitrogen molybdenum fullerene lubricant. However, on the one hand, the antiwear additive product has an unremarkable improving effect on the lubricity of low-sulfur diesel oil. On the other hand, as one of the components of the additive is organic nitrogen molybdenum fullerene, the preparation cost of the antiwear additive product is greatly increased, and the industrial prospect is poor.

EP patent application EP1404726A2 discloses a polymeric lubricity modifier for improving lubricity of low-sulfur diesel oil. The additive is composed of polyisobutenyl succinic anhydride derivatives with a number average molecular weight of 300-1000 or polyisobutenyl succinic anhydride derivatives with a number average molecular weight of 2000-20000. However, the polymerization reaction conditions are relatively harsh, and the requirements for production equipment are relatively high. In addition, the polymerization reaction is not easy to control. If the molecular weight of the polymer is too high, it will be difficult to be miscible with diesel oil, and it will be difficult to show good lubricity improving performance.

Therefore, there is still a need for a lubricity modifier having good stability and lubricity improving performance.

### Disclosure of the Invention

The present application provides a lubricity modifier product capable of improving lubricity of oil products, which can show good lubricity improving performance in engine fuel oil, lubricating oil, etc., and has good stability.

In order to achieve the above object, in one aspect, the present application provides a lubricity modifier suitable for improving lubricity of oil products, comprising 50-100 wt% of a compound having a structure represented by following formula (I), 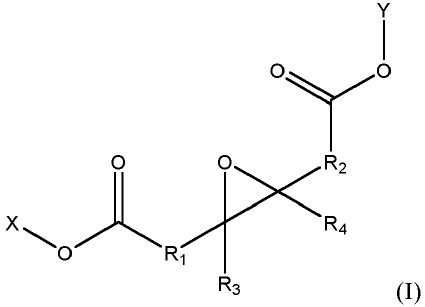
wherein, groups R₁ and R₂ are each independently a single bond or a substituted or unsubstituted C₁-C₂₀ hydrocarbylene group;
groups R₃ and R₄ are each independently hydrogen or a substituted or unsubstituted C₁-C₁₀ hydrocarbyl group;
groups X and Y are each independently hydrogen or a substituted or unsubstituted C₁-C₅₀ hydrocarbyl group, and at least one of X and Y is hydrogen,
wherein the "substituted" refers to being substituted with at least one group selected from the group consisting of hydroxyl, carboxyl, ester group, hydrocarbyloxy, primary amino, secondary amino, amido, and aminoacyl.

In another aspect, the present application provides a lubricating oil composition, comprising a base oil for lubricating oil and 0.01-30 wt%, based on mass of the base oil for lubricating oil, of the compound having the structure of formula (I), wherein the compound is used as a lubricity modifier.

In another aspect, the present application provides a method for improving lubricity of a lubricating oil, comprising adding to the lubricating oil 0.01-30 wt%, based on mass of a base oil for lubricating oil in the lubricating oil, of the compound having the structure of formula (I) as a lubricity modifier.

In a further aspect, the present application provides an engine fuel oil composition, comprising a liquid base fuel and 10-5000 mg/kg, based on mass of the base fuel, of the compound having the structure of formula (I), wherein the compound is used as a lubricity modifier.

In a further aspect, the present application provides a method for improving lubricity of an engine fuel oil, comprising adding to the engine fuel oil 10-5000 mg/kg, relative to mass of a base fuel in the engine fuel oil, of the compound having the structure of formula (I) as a lubricity modifier.

In yet another aspect, the present application provides a method of using the compound having the structure of formula (I) as a lubricity modifier for oil products, comprising a step of adding the compound to an oil product.

In yet another aspect, the present application provides an additive composition for oil products, comprising from 10 wt% to less than 100 wt% of the compound having the structure of formula (I), and from greater than 0 wt% to 90 wt% of one or more additive for improving properties of oil products, wherein the compound is used as a lubricity modifier.

The inventor of the present application unexpectedly found in their research that the compound having the structure of formula (I) can be used in oil products as a lubricity modifier, and has the following advantages compared with the lubricity modifiers of the prior art:
(1) the lubricity of an oil product can be significantly improved by adding a small amount of the compound into the oil product;
(2) the compound has good stability, is not easy to oxidize and/or deteriorate, and can maintain good lubricity improving effect for a long time;
(3) the synthesis process is simple, the synthesis steps are short and easy to operate, the reaction conditions are mild, and the reaction process is easy to control;
(4) the raw materials are widely sourced and cheap, resulting in relatively low production costs.

Additional features and advantages of the application will be set forth in the detailed description which follows.

### Brief Description of the Drawings

The drawings, constituting a part of the present description, are provided to help the further understanding of the present application, and should not be considered to be limiting. The present application can be interpreted with reference to the drawings in combination with the detailed description hereinbelow. In the drawings:
FIG. 1 shows a wear scar graph of blank diesel oil without a lubricity modifier; and
FIG. 2 shows a wear scar graph measured in Example 3 after adding a lubricity modifier of the present application at a dosage of 200 mg/kg to the blank diesel oil.
FIG. 3 shows an infrared spectrum of monoisooctyl epoxysuccinate product obtained in Preparation Example 1.

### Detailed Description of the Invention

The present application will be further described hereinafter in detail with reference to specific embodiments thereof and the accompanying drawings. It should be noted that the specific embodiments of the present application are provided for illustration purpose only, and are not intended to be limiting in any manner.

Any specific numerical value, including the endpoints of a numerical range, described in the context of the present application is not restricted to the exact value thereof, but should be interpreted to further encompass all values close to said exact value, for example all values within ±5% of said exact value. Moreover, regarding any numerical range described herein, arbitrary combinations can be made between the endpoints of the range, between each endpoint and any specific value within the range, or between any two specific values within the range, to provide one or more new numerical range(s), where these new numerical range(s) should also be deemed to have been specifically described in the present application.

Unless otherwise stated, the terms used herein have the same meaning as commonly understood by those skilled in the art; and if the terms are defined herein and their definitions are different from the ordinary understanding in the art, the definition provided herein shall prevail.

In the present application, the term "hydrocarbyl group" refers to a group obtained by removing one hydrogen atom from a saturated or unsaturated hydrocarbon, and includes various aliphatic hydrocarbyl groups, alicyclic hydrocarbyl groups and aromatic hydrocarbyl groups, such as alkyl, cycloalkyl, aryl, aralkyl, alkaryl, alkenyl, cycloalkenyl, and the like.

In the present application, the term "hydrocarbylene group" refers to a group obtained by removing two hydrogen atoms from a saturated or unsaturated hydrocarbon, and includes various aliphatic hydrocarbylene groups, alicyclic hydrocarbylene groups and aromatic hydrocarbylene groups, such as alkylene, cycloalkylene, arylene, aralkylene, alkarylene, alkenylene, cycloalkenylene, and the like.

In the present application, the term "substituted" means that the group is substituted with one or more substituent(s), which may be various substituents commonly found in the field of organic synthesis, preferably selected from the group consisting of hydroxyl, carboxyl, ester group, hydrocarbyloxy, primary amino, secondary amino, amido and aminoacyl, more preferably selected from the group consisting of hydroxyl, carboxyl, ester group and hydrocarbyloxy.

In the present application, the term "ester group" refers to a group having a structure represented by the formula -COOR, wherein R is a substituted or unsubstituted hydrocarbyl group, for example, a C₁₋₄ hydrocarbyl group, such as methyl, ethyl, propyl, butyl, etc.

In the present application, the term "hydrocarbyloxy" refers to a group having a structure represented by the formula -O-R, wherein R is a substituted or unsubstituted hydrocarbyl group, for example, a C₁₋₄ hydrocarbyl group, such as methyl, ethyl, propyl, butyl, etc.

In the present application, the term "half-esterification reaction" refers to a reaction of partially esterifying an organic dicarboxylic acid to prepare a dicarboxylic acid monoester.

In the context of the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to the person skilled in the art that such a combination is obviously unreasonable.

All of the patent and non-patent documents cited herein, including but not limited to textbooks and journal articles, are hereby incorporated by reference in their entirety.

In a first aspect, the present application provides a lubricity modifier suitable for improving lubricity of oil products, comprising 50-100 wt%, preferably 60-100 wt%, more preferably 70-100 wt%, further preferably 80-100 wt%, particularly preferably 90-100 wt% of a compound having a structure represented by following formula (I),

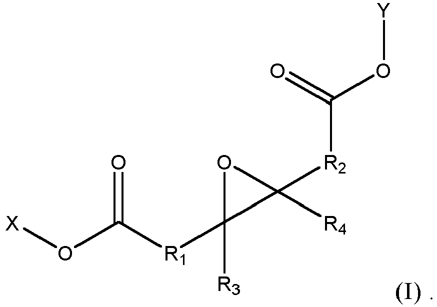

Depending on the actual situation, in addition to the compound having the structure of formula (I), minor amounts of additional components, such as diesel oil, organic solvents, unreacted raw materials for preparation (such as alcohols or phenols), reaction by-products, and the like, may be comprised in the lubricity modifier of the present application. However, the total amount of these additional components is no more than 50 wt%, preferably no more than 30 wt%, more preferably no more than 10 wt%, for example no more than 5 wt%, of the total weight of the lubricity modifier composition for fuel oil.

In a second aspect, the present application provides a lubricating oil composition, comprising a base oil for lubricating oil and, based on mass of the base oil for lubricating oil, 0.01-30 wt%, preferably 0.05-20 wt%, further preferably 0.1-10 wt% of the compound having the structure of formula (I), wherein the compound is used as a lubricity modifier.

In a third aspect, the present application provides a method for improving lubricity of a lubricating oil, comprising adding to the lubricating oil 0.01-30 wt%, preferably 0.05-20 wt%, further preferably 0.1-10 wt%, based on mass of a base oil for lubricating oil in the lubricating oil, of the compound having the structure of formula (I) as a lubricity modifier.

The base oil for lubricating oil suitable for use in the present application is not strictly limited and can be those conventionally used in the field of lubricating oils, including, for example, but not limited to, mineral base oils, synthetic base oils, and bio-base oils. As specific examples, the synthetic oils generally include silicone oils, polyethers, poly α-olefins, polyphenylene ethers, phosphates, and the like; the bio-base oils generally include natural vegetable oils, synthetic esters, and the like.

In a fourth aspect, the present application provides an engine fuel oil composition, comprising a liquid base fuel and 10-5000 mg/kg, preferably 50-1000 mg/kg, further preferably 100-500 mg/kg, based on mass of the base fuel, of the compound having the structure of formula (I), wherein the compound is used as a lubricity modifier.

In a fifth aspect, the present application provides a method for improving lubricity of an engine fuel oil, comprising adding to the engine fuel oil 10-5000 mg/kg, preferably 50-1000 mg/kg, further preferably 100-500 mg/kg, relative to mass of a base fuel in the engine fuel oil, of the compound having the structure of formula (I) as a lubricity modifier.

The base fuel suitable for use in the present application is not strictly limited and can be those conventionally used in the field of engine fuel oils, including all of petroleum-based source fuels, such as middle distillate fuels, diesel fuels, gasoline, jet fuels, kerosene, marine oils, and other low-sulfur fuels, high asphaltene fuels, liquefied petroleum gas fuels, and the like; bio-renewable fuels, such as biodiesel fuels, biomass-to-liquid (BTL) fuels, and the like; synthetic fuels, such as gas-to-liquid (GTL) fuels and other synthetic fuels, and the like; coal-based source fuels, such as alcohols, ethers, Fischer-Tropsch synthetic fuels, coal-to-liquid (CTL) fuels, fuels derived from coal and/or petroleum coke; other fuels, such as genetically engineered biofuels, and crops and extract fuels thereof, and the like. Preferably, the base fuel is selected from the group consisting of gasoline fuels and diesel fuels.

In a sixth aspect, the present application provides a method of using the compound having the structure of formula (I) as a lubricity modifier for oil products, comprising a step of adding the compound to an oil product.

In a preferred embodiment, the oil product is a lubricating oil or an engine fuel oil.

In a seventh aspect, the present application provides an additive composition for oil products, comprising from 10 wt% to less than 100 wt%, preferably 30-90 wt%, more preferably 50-90 wt%, of the compound having the structure of formula (I), and from greater than 0 wt% to 90 wt%, preferably 10-70 wt%, more preferably 10-50 wt%, of one or more additive for improving properties of oil products, wherein the compound having the structure of formula (I) is used as a lubricity modifier.

The additive for improving properties of oil products, suitable for use in the present application, is not strictly limited and can be those conventionally used in the field of oil products, as long as it does not adversely interact with the compound having the structure of formula (I). As specific examples, there may be mentioned detergent dispersants, metal deactivators, corrosion inhibitors, antioxidants, rust inhibitors, cetane improvers, pour point depressants, combustion promoters, or combinations thereof.

According to the present application, in the compound having the structure of formula (I), groups R₁ and R₂ are each independently a single bond or a substituted or unsubstituted C₁-C₂₀ hydrocarbylene group;
groups R₃ and R₄ are each independently hydrogen or a substituted or unsubstituted C₁-C₁₀ hydrocarbyl group;
groups X and Y are each independently hydrogen or a substituted or unsubstituted C₁-C₅₀ hydrocarbyl group, and at least one of X and Y is hydrogen,
wherein the "substituted" refers to being substituted with at least one group selected from the group consisting of hydroxyl, carboxyl, ester group, hydrocarbyloxy, primary amino, secondary amino, amido, and aminoacyl, preferably selected from the group consisting of hydroxyl, carboxyl, ester group, and alkoxy.

The inventors of the present application have unexpectedly found in their research that a compound having a structure represented by formula (I) can function as a lubricity modifier for oil products, exhibits good lubricity improving performance in various oil products (such as engine fuel oil, lubricating oil, etc.), and has good stability, thereby obtaining the various embodiments described above in the present application.

In a preferred embodiment, in the compound having the structure of formula (I), groups R₁ and R₂ are each independently a single bond or a substituted or unsubstituted C₁-C₁₀ alkylene group; groups R₃ and R₄ are each independently hydrogen or a substituted or unsubstituted C₁-C₅ hydrocarbyl group; groups X and Y are each independently hydrogen or a substituted or unsubstituted C₄-C₃₀ hydrocarbyl group, and at least one of X and Y is hydrogen, wherein the "substituted" refers to being substituted with at least one group selected from hydroxyl, carboxyl, ester group, and hydrocarbyloxy group.

In a further preferred embodiment, in the compound having the structure of formula (I), groups R₁ and R₂ are each independently a single bond or a substituted or unsubstituted C₁-C₄ alkylene group; groups R₃ and R₄ are each independently hydrogen or a substituted or unsubstituted C₁-C₂ hydrocarbyl group; groups X and Y are each independently hydrogen or a substituted or unsubstituted C₄-C₂₀ hydrocarbyl group, and at least one of X and Y is hydrogen, wherein the "substituted" refers to being substituted with at least one group selected from hydroxyl, carboxyl, ester group, and hydrocarbyloxy group.

In a still further preferred embodiment, in the compound having the structure of formula (I), groups R₁ and R₂ are single bonds and groups R₃ and R₄ are each independently hydrogen or C₁-C₂ alkyl group.

In a still further preferred embodiment, one of X and Y is hydrogen and the other is a substituted or unsubstituted C₄-C₂₀ hydrocarbyl group.

In certain particularly preferred embodiments, in the compound having the structure of formula (I), groups R₁ and R₂ are single bonds and groups R₃ and R₄ are hydrogen, then the compound has a structure represented by following formula (II):

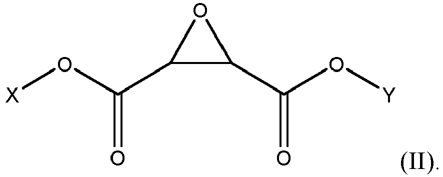

In a further preferred embodiment, in the formula (II), group X is hydrogen and group Y is a substituted or unsubstituted C₄-C₂₀ hydrocarbyl group.

In other particularly preferred embodiments, in the compound having the structure of formula (I), group R₁ is a single bond, R₂ is methylene, and groups R₃ and R₄ are hydrogen, then the compound has a structure represented by following formula (III-1) or (III-2):

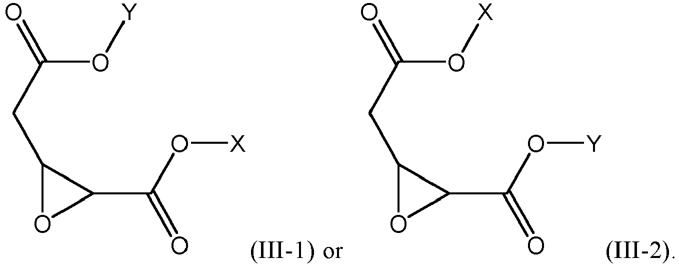

In a further preferred embodiment, in the formula (III-1) or (III-2), group X is hydrogen and group Y is a substituted or unsubstituted C₄-C₂₀ hydrocarbyl group.

In still further preferred embodiments, in the formula (II), (III-1) or (III-2), when group Y is a C₄-C₂₀ linear or branched alkyl group, it may be selected from, for example, n-butyl, iso-butyl, n-pentyl, iso-pentyl, n-hexyl, iso-hexyl, n-heptyl, iso-heptyl, n-octyl, iso-octyl, n-nonyl, iso-nonyl, n-decyl, iso-decyl, n-dodecyl, iso-dodecyl, n-tetradecyl, iso-tetradecyl, n-hexadecyl, iso-hexadecyl, n-octadecyl, iso-octadecyl, n-eicosyl, iso-eicosyl, and the like; alternatively, when group Y is a C₄-C₂₀ linear or branched alkenyl group, it may be selected from, for example, n-butenyl, iso-butenyl, n-pentenyl, iso-pentenyl, n-hexenyl, iso-hexenyl, n-heptenyl, iso-heptenyl, n-octenyl, iso-octenyl, n-nonenyl, iso-nonenyl, n-decenyl, iso-decenyl, n-dodecenyl, iso-dodecenyl, n-tetradecenyl, iso-tetradecenyl, n-hexadecenyl, iso-hexadecenyl, n-octadecenyl, iso-octadecenyl, n-eicosenyl, iso-eicosenyl, and the like; when group Y is a C₄-C₂₀ cycloalkyl group, it may be selected from, for example, cyclopentyl, cyclohexyl, methylcyclopentyl, methylcyclohexyl, etc.; when group Y is a C₄-C₂₀ cycloalkenyl group, it may be selected from, for example, cyclopentenyl, cyclohexenyl, methylcyclopentenyl, methylcyclohexenyl, etc.; when group Y is a substituted C₄-C₂₀ hydrocarbyl group, the substituent(s) thereon may be an oxygen-containing functional group, such as hydroxyl (-OH), carboxyl (-COOH), ester group (-COOR), and hydrocarbyloxy (-O-R), or a nitrogen-containing functional group, such as primary amino (-NH₂), secondary amino (-NH-), amido (-C(=O)-NH-), aminoacyl (-NH-C(=O)-), or the like. As specific examples of the substituted C₄-C₂₀ hydrocarbyl group, there may be mentioned -C₈H₁₆OH, -C₈H₁₆COOH, - C₈H₁₆COOCH=CH₂, -C₂H₄OC₈H₁₇, -C₁₇H₃₄COOCH₃, -C₁₇H₃₄COOH, -C₁₇H₃₂COOH, -C₁₇H₃₂COOCH₃, -C₁₈H₃₂COOCH₃, -C₁₈H₃₄COOCH₃, -CH₂CH₂OCH₂CH₂OCH₃, - C₈H₁₆NH₂, -C₂H₄NH(O=C)C₈H₁₇ and the like.

As specific examples, the compound having the structure of formula (II) may be selected from: mono-n-butyl epoxysuccinate, mono-iso-butyl epoxysuccinate, mono-n-pentyl epoxysuccinate, mono-iso-pentyl epoxysuccinate, mono-n-octyl epoxy succinate, mono-iso-octyl epoxysuccinate, mono-n-nonyl epoxysuccinate, mono-iso-nonyl epoxysuccinate, mono-lauryl epoxysuccinate, mono-octadecyl epoxy succinate, mono-oleyl epoxysuccinate, etc.; mono-cyclopentyl epoxysuccinate, mono-cyclohexyl epoxysuccinate, mono-poly(ethylene glycol) mono-methyl ether epoxysuccinate, mono-poly(ethylene glycol) epoxysuccinate, poly(propylene glycol) epoxysuccinate, poly(propylene glycol) mono-butyl ether epoxysuccinate, epoxysuccinic acid monoester group-containing ricinoleic acid (3-(((17-carboxyheptadec-9-en-7-yl)oxy)carbonyl)oxirane-2-carboxylic acid), epoxysuccinic acid monoester group-containing methyl ricinoleate (3-(((18-methoxy-18-oxooctadec-9-en-7-yl)oxy)carbonyl)oxirane-2-carboxylic acid), 12-epoxysuccinic acid monoester group-containing stearic acid (3-(((17-carboxyheptadec-7-yl)oxy)carbonyl)oxirane-2-carboxylic acid), 12-epoxysuccinic acid monoester group-containing methyl stearate (3-(((18-methoxy-18-oxooctadec-7-yl)oxy)carbonyl)oxirane-2-carboxylic acid), or combinations thereof.

As specific examples, the compound having the structure of formula (III-1) or (III-2) may be selected from: epoxidized mono-n-butyl glutaconate, epoxidized mono-iso-butyl glutaconate, epoxidized mono-n-pentyl glutaconate, epoxidized mono-iso-pentyl glutaconate, epoxidized mono-n-octyl glutaconate, epoxidized mono-iso-octyl glutaconate, epoxidized mono-n-nonyl glutaconate, epoxidized mono-iso-nonyl glutaconate, epoxidized mono-lauryl glutaconate, epoxidized mono-octadecyl glutaconate, epoxidized mono-oleyl glutaconate, etc.; epoxidized mono-cyclopentyl glutaconate, epoxidized mono-cyclohexyl glutaconate, epoxidized mono-poly(ethylene glycol) mono-methyl ether glutaconate, epoxidized mono-poly(ethylene glycol) glutaconate, epoxidized poly(propylene glycol) glutaconate, epoxidized poly(propylene glycol) mono-butyl ether glutaconate, epoxidized glutaconic acid monoester group-containing ricinoleic acid (2-(3-(((17-carboxyheptadec-9-en-7-yl)oxy)carbonyl)oxirane-2-yl)acetic acid / 3-(2-((17-carboxyheptadec-9-en-7-yl)oxy)-2-oxoethyl)oxirane-2-carboxylic acid), epoxidized glutaconic acid monoester group-containing methyl ricinoleate (2-(3-(((18-methoxy-18-oxooctadec-9-en-7-yl)oxy)carbonyl)oxiran-2-yl)acetic acid / 3-(2-((18-methoxy-18-oxooctadec-9-en-7-yl)oxy)-2-oxoethyl)oxirane-2-carboxylic acid), 12-epoxidized glutaconic acid monoester group-containing stearic acid (2-(3-(((17-carboxyheptadec-7-yl)oxy)carbonyl)oxirane-2-yl)acetic acid / 3-(2-((17-carboxyheptadec-7-yl)oxy)-2-oxoethyl)oxirane-2-carboxylic acid), 12-epoxidized glutaconic acid monoester group-containing methyl stearate (2-(3-(((18-methoxy-18-oxooctadec-7-yl)oxy)carbonyl)oxiran-2-yl)acetic acid / 3-(2-((18-methoxy-18-oxooctadec-7-yl)oxy)-2-oxoethyl)oxirane-2-carboxylic acid), or combinations thereof.

According to the present application, the compound having the structure of formula (I) may be prepared by a method comprising steps of:
(1) carrying out half-esterification reaction on unsaturated dicarboxylic acid having a structure represented by following formula (IV) or anhydride thereof and a hydroxyl-containing compound X-OH and/or Y-OH to obtain a reaction intermediate, 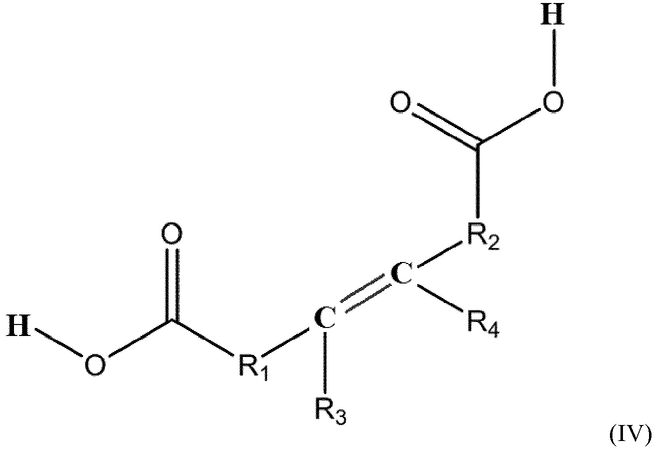 wherein, groups R₁, R₂, R₃, R₄, X and Y are as defined above;
(2) carrying out epoxidation reaction on the reaction intermediate obtained in step (1) in the presence of peroxide to obtain the compound having the structure represented by formula (I).

Preferably, in step (1), the temperature of the half-esterification reaction is 20-200°C, preferably 50-150°C, more preferably 75-120°C; the reaction time is 0.5-20 hours, preferably 1-10 hours, more preferably 1-4 hours; the molar ratio of the unsaturated dicarboxylic acid or anhydride thereof to the hydroxyl-containing compound is from 1:2 to 1:0.1, preferably more than 1:2 to 1:0.5, more preferably more than 1:2 to 1:1.

Preferably, in step (2), the temperature of the epoxidation reaction is 0-180°C, preferably 20-150°C, more preferably 30-100°C; the reaction time is 0.5-20 hours, preferably 1-10 hours; the molar ratio of the reaction intermediate to the peroxide is 1:0.1-10, preferably 1:0.5-5, more preferably 1:1-3.

According to the present application, the compound having the structure of formula (I) may also be prepared by a method comprising steps of:
(a) carrying out epoxidation reaction on unsaturated dicarboxylic acid having a structure represented by formula (IV) or anhydride thereof in the presence of peroxide to obtain a reaction intermediate; 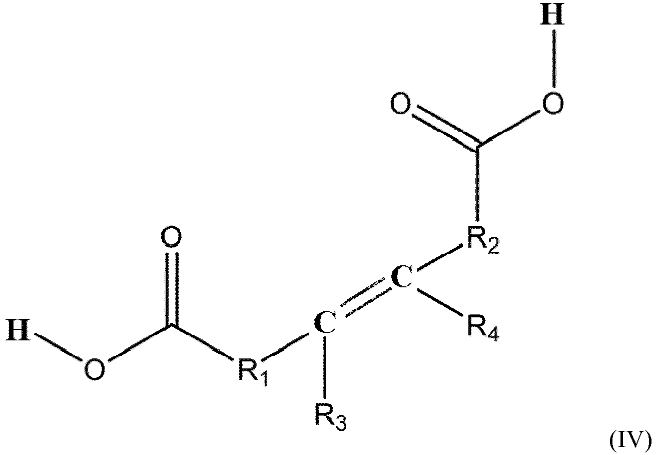
(b) carrying out half-esterification reaction on the reaction intermediate obtained in step (a) and a hydroxyl-containing compound X-OH and/or Y-OH to obtain the compound having the structure represented by formula (I),
   wherein, groups R₁, R₂, R₃, R₄, X and Y are as defined above.

Preferably, in step (a), the temperature of the epoxidation reaction is 0-180°C, preferably 20-150°C, more preferably 30-100°C; the reaction time is 0.5-30 hours, preferably 1-20 hours, more preferably 6-10 hours; the molar ratio of the unsaturated dicarboxylic acid or anhydride thereof to the peroxide is 1:0.1-10, preferably 1:0.5-5, more preferably 1:1-3.

Preferably, in step (b), the temperature of the half-esterification reaction is 20-200°C, preferably 50-150°C, more preferably 75-120°C; the reaction time is 0.5-20 hours, preferably 1-10 hours, more preferably 1-4 hours; the molar ratio of the reaction intermediate to the hydroxyl-containing compound is from 1:2 to 1:0.1, preferably greater than 1:2 to 1:0.5, more preferably greater than 1:2 to 1:1.

According to the present application, in the above two preparation methods, the unsaturated dicarboxylic acid having the structure of formula (IV) or anhydride thereof includes, but is not limited to: cis-butenedioic acid (maleic acid), trans-butenedioic acid (fumaric acid), cis-butenedioic anhydride (maleic anhydride), trans-butenedioic anhydride (fumaric anhydride), 2,3-dimethylmaleic acid, 2,3-dimethylmaleic anhydride, 2-methylmaleic acid, 2-methylmaleic anhydride, glutaconic acid, glutaconic anhydride, 3-methylglutaconic acid, decenedioic acid, decenedioic anhydride, hexadienedioic acid, dodecenedioic acid, octadecenedioic acid, eicosendioic acid, and the like, or various combinations thereof.

According to the present application, in the above two preparation methods, the hydroxyl-containing compound may be selected from C₁-C₅₀, preferably C₄-C₃₀, more preferably C₄-C₂₀, fatty alcohols, hydroxy fatty acids, hydroxy fatty acid esters, hydroxy fatty amines, hydroxy amides, ether alcohols, phenolic compounds, or combinations thereof. As specific examples, the fatty alcohols include, but are not limited to, n-butanol, iso-butanol, n-pentanol, iso-pentanol, n-octanol, iso-octanol, n-nonanol, iso-nonanol, lauryl alcohol, tetradecanol, octadecanol, oleyl alcohol, eicosanol, pentenol, hexenol, polyethylene glycol, polypropylene glycol, cyclohexanol, cyclopentanol, cyclohexenol, cyclopentenol, and the like; the hydroxy fatty acids include, but are not limited to, ricinoleic acid, 12-hydroxystearic acid, and the like; the hydroxy fatty acid ester includes, but is not limited to, methyl 12-hydroxystearate, methyl ricinoleate, ethyl ricinoleate, butyl ricinoleate, biodiesel containing a component of methyl ricinoleate, and the like; the hydroxy fatty amines include, but are not limited to, N-methyl dioctanolamine, octanolamine, and the like; the hydroxyamides include, but are not limited to, octyl hydroxyacetamide, and the like; the ether alcohols include, but are not limited to, polyethylene glycol monomethyl ether, polypropylene glycol monobutyl ether, and the like; the phenolic compounds include, but are not limited to, pentylphenol, heptylphenol, octylphenol, nonylphenol, decylphenol, dodecylphenol, pentadecylphenol, alkylphenol ethoxylates, and the like.

According to the present application, examples of peroxides suitable for use in the above two preparation methods include, but are not limited to, hydrogen peroxide, peroxy acids, alkyl peroxides. As a specific example, the hydrogen peroxide may be a hydrogen peroxide solution with a mass fraction of 0. 1-50%, preferably a hydrogen peroxide solution of 10-40%; the alkyl peroxides include, but are not limited to, dimethyl peroxide, diethyl peroxide, t-butyl methyl peroxide, di-sec-butyl peroxide, di-t-butyl peroxide, t-butyl t-amyl peroxide, t-butyl cumyl peroxide, 2,5-dimethyl-2,5-bis(t-butylperoxy)hexane, 2,5-dimethyl-2,5-bis(t-butylperoxy)-3-hexyne, dicumyl peroxide, 1,4-bis(2-t-butylperoxyisopropyl)benzene, cyclohexyl hydroperoxide, cumyl hydroperoxide, and the like.

According to the present application, the epoxidation reaction step of the above two preparation methods is preferably carried out in the presence of a catalyst, and the catalysts used may be those conventionally used in the art, including, but not limited to, inorganic acid catalyst systems, cation exchange resin catalyst systems, organic acid catalyst systems, aluminum sulfate catalyst systems, m-chloroperoxybenzoic acid catalyst systems, t-butyl hydroperoxide catalyst systems, enzyme catalyst systems, titanium-silicon molecular sieve-based catalysts, heteropoly compound catalyst systems, metal complex catalyst systems, methyltrioxorhenium catalyst systems, metal-organic complex catalyst systems, and the like, preferably tungsten compound, titanium-silicon molecular sieve-based catalyst, and phosphorus compound catalyst systems, such as tungstic acid, sodium tungstate, aminomethylphosphonic acid, phosphoric acid, sodium dihydrogen phosphate, potassium dihydrogen phosphate, titanium-silicon molecular sieves having an MFI topology, and the like. Preferably, the total amount of catalyst used is 0.01-10.0%, preferably 0.05-5% of the total mass of the reactants.

According to the present application, the epoxidation reaction step of the above two preparation methods is optionally and preferably carried out in the presence of an oxygen carrier, examples of which include, but are not limited to, formic acid, acetic acid, and the like, preferably formic acid. Preferably, the oxygen carrier is used in an amount of 0.01%-70%, preferably 1%-50%, more preferably 10%-30% of the total mass of the reactants.

According to the present application, in the preferred embodiment of the above two preparation methods, the peroxide and the catalyst may be added in any one of the following three modes: (i) the peroxide can be dropwise added into the reactant solution together with the catalyst, wherein the dropwise adding rate is controlled to be 1-20 drops/second, preferably 1-10 drops/second, more preferably 1-5 drops/second; (ii) firstly adding the catalyst into the reaction system, and then independently dropwise adding the peroxide into the reactant solution, wherein the dropwise adding rate is controlled to be 1-20 drops/second, preferably 1-10 drops/second, more preferably 1-5 drops/second; or (iii) adding the peroxide and the catalyst into the reaction system together, and carrying out the reaction by "one-pot method", preferably (ii) reactant feeding mode.

According to the present application, in the above two preparation methods, a solvent, which may be a polar solvent or a nonpolar solvent, is optionally used in the reaction system. Preferably, the amount of the solvent used may be 1-300%, preferably 10-150%, further preferably 30-100% by mass ratio, relative to mass of the compound of formula (III). As specific examples, the solvent may include, but is not limited to, aromatic hydrocarbons, mineral spirits, alkylene oxides, cycloalkanes, n-alkanes, isoparaffins, petroleum ethers, and the like, or combinations thereof; preferably selected from the group consisting of aromatic hydrocarbons, mineral spirits, n-pentane, n-hexane, petroleum ethers, carbon disulfide, carbon dioxide, methyl formate, methyl tert-butyl ether, dimethoxyethane, dimethyl ether, dimethoxymethane, ethyl methyl ether, diethyl ether, acetone, and combinations thereof.

According to the present application, the above two preparation methods can be carried out under normal pressure condition or under pressurized condition, and the pressure may be in the range of 0.01-10 MPa, and the reaction is preferably carried out under normal pressure condition.

According to the present application, in the above two preparation methods, after the reaction is completed, the product may or may not be purified.

### Examples

The present application will be further illustrated with reference to the following examples, but the present application is not limited thereto.

Unless indicated to the contrary, the reagents used in the following preparations and examples are commercially available products with purity of analytical grade.

In the following examples and comparative examples, the lubricity of diesel oil was evaluated by measuring the Wear Scar Diameter (WSD) at 60°C on a High-Frequency Reciprocating Rig (HFRR, PCS Instruments, UK) according to ISO 12156-1 method and the influence of temperature and humidity was corrected to obtain the reported results of Wear Scar Diameter WS1.4.

The following preparation examples illustrate the preparation method of the compound having the structure represented by formula (I).

### Preparation Example 1

49 g (0.5 mol) of maleic anhydride (analytical grade, Beijing InnoChem Science & Technology Co., Ltd.) and 65.5 g (0.5 mol) of iso-octanol (analytical grade, Beijing InnoChem Science & Technology Co., Ltd.) were charged into a reactor equipped with an electric stirrer, a thermometer, a reflux condenser, a dropping funnel and a nitrogen catheter. Nitrogen was introduced for 5-10 minutes while maintaining a weak nitrogen flow during the reaction. The mixture was heated under stirring to 100°C. The molar ratio of maleic anhydride to iso-octanol was about 1:1, and the reaction time was 1.5 hours. Mono-iso-octyl maleate reaction intermediate was prepared to give about 113.6 g of the reaction intermediate.

113.6 g of the reaction intermediate was charged into a reactor with a three-neck flask, and 12 g of sodium tungstate was added as a catalyst, wherein the mass of sodium tungstate accounted for about 10% of that of the reaction intermediate. The mixture was heated to 65°C, and 15 g of formic acid (analytical grade, Beijing InnoChem Science & Technology Co., Ltd.) was added as an oxygen carrier. Under stirring, 85 g of hydrogen peroxide solution (mass fraction: 30%, Shanghai Aladdin Biochemical Technology Co., Ltd.) was slowly dropwise added with the molar ratio of mono-iso-octyl maleate to hydrogen peroxide of about 1:3. The dropwise adding rate was controlled to be 2 drops/second, and the reaction was conducted for 3 hours. The product was cooled, dried and filtered to give about 196.4 g of mono-iso-octyl epoxysuccinate product. The reaction process is shown as Reaction Formula 1.

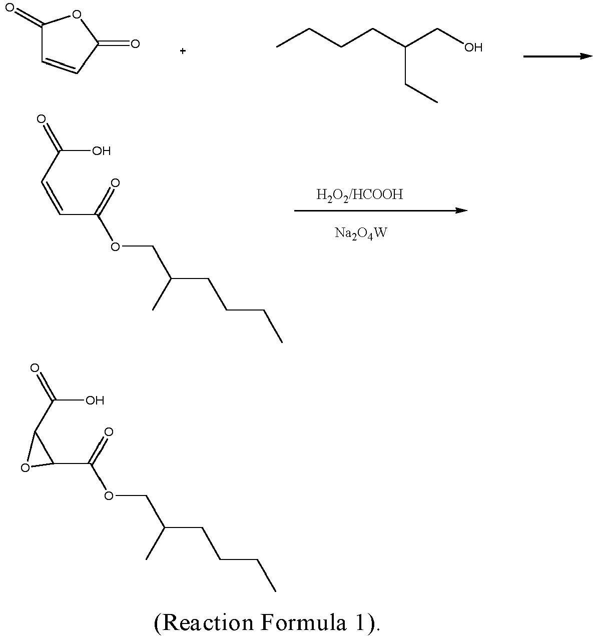

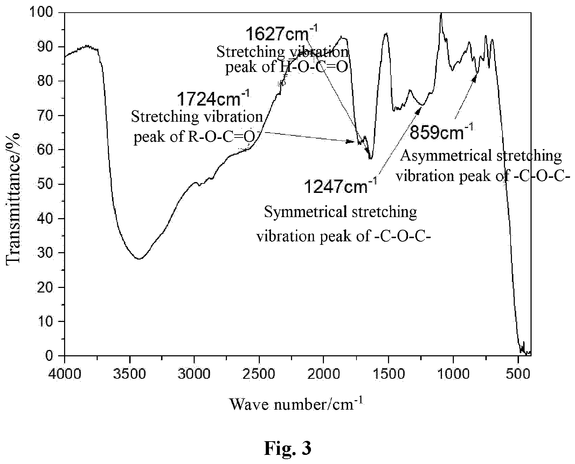

The infrared spectrum of the product is shown as FIG. 3, wherein the wave number 1724 cm⁻¹ corresponds to the stretching vibration peak of -R-O-C=O (ester group), the wave number 1627 cm⁻¹ corresponds to the stretching vibration peak of H-O-C=O- (carboxyl group), the wave number 1247 cm⁻¹ corresponds to the symmetrical stretching vibration peak of -C-O-C-, and the wave number 859 cm⁻¹ corresponds to the asymmetrical stretching vibration peak of -C-O-C-. This indicates that mono-iso-octyl epoxysuccinate product was prepared by the above process.

### Preparation Example 2

49 g (0.5 mol) of maleic anhydride (analytical grade, Beijing InnoChem Science & Technology Co., Ltd.) and 61.3 g (0.6 mol) of n-hexanol (analytical grade, Beijing InnoChem Science & Technology Co., Ltd.) were charged into a reactor equipped with an electric stirrer, a thermometer, a reflux condenser, a dropping funnel and a nitrogen catheter. Nitrogen was introduced for 5-10 minutes while maintaining a weak nitrogen flow during the reaction. The mixture was heated under stirring to 85°C. The molar ratio of maleic anhydride to n-hexanol was about 1:1.2, and the reaction time was 1.5 hours. Mono-n-hexyl maleate reaction intermediate was prepared to give about 109.6 g of the reaction intermediate.

109.6 g of the reaction intermediate was charged into a reactor with a three-neck flask, and 11 g of titanium-silicon molecular sieve was added as a catalyst, wherein the mass of titanium-silicon molecular sieve catalyst accounted for about 10% of that of the reaction intermediate. The mixture was heated to 65°C, and under stirring, 37.5 g of hydrogen peroxide solution (mass fraction: 30%, Shanghai Aladdin Biochemical Technology Co., Ltd.) was slowly dropwise added with the molar ratio of mono-n-hexyl maleate to hydrogen peroxide of about 1:2. The dropwise adding rate was controlled to be 2 drops/second, and the reaction was conducted for 6 hours. The product was cooled, dried and filtered to give about 139.3 g of mono-n-hexyl epoxysuccinate product. The reaction process is shown as Reaction Formula 2.

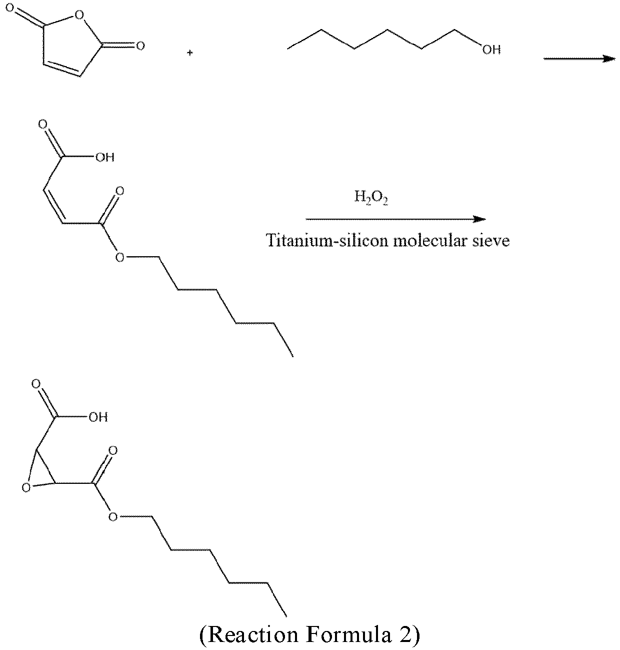

### Preparation Example 3

49 g (0.5 mol) of maleic anhydride (analytical grade, Beijing InnoChem Science & Technology Co., Ltd.) was charged into a three-neck flask reactor equipped with a stirrer, a dropping device, a reflux condenser, a thermometer and a heating device, and 2.5 g of sodium tungstate (analytical grade, Beijing InnoChem Science & Technology Co., Ltd.) was added. Nitrogen was introduced for 5-10 minutes while maintaining a weak nitrogen flow during the reaction. The mixture was heated to 70°C until the maleic anhydride was completely melted into liquid. 51 g (1.5 mol) of 30% hydrogen peroxide solution (Beijing InnoChem Science & Technology Co., Ltd.) was weighed and dropwise added into the maleic anhydride solution. The dropwise adding rate was controlled to be about 2-5 drops/second, keeping the temperature to be 70°C. The reaction was conducted for 6 hours to prepare epoxy succinic anhydride reaction intermediate, which was subjected to filtration and standing to give about 96.5 g of the product.

96.5 g of epoxy succinic anhydride was charged into a three-neck flask reactor equipped with a stirrer, a dropping device, a reflux condenser, a thermometer and a heating device, and 183 g of iso-nonyl alcohol (analytical grade, Beijing YinuoKai science and technology Co., Ltd.) was weighed and added into the reaction system. The mixture was heated to 150°C, and the reaction was conducted for 2.5 hours to prepare mono-iso-nonyl epoxysuccinate. The product was subjected to rotary distillation treatment to purify mono-iso-nonyl epoxysuccinate to give about 267.9 g of the product, i.e. the desired mono-iso-nonyl epoxysuccinate.

### Preparation Example 4

49 g (0.5 mol) of maleic anhydride (analytical grade, Beijing InnoChem Science & Technology Co., Ltd.) and 157.3 g (0.5 mol) of methyl 12-hydroxystearate (99%, Shanghai Aladdin Biochemical Technology Co., Ltd.) were charged into a reactor equipped with an electric stirrer, a thermometer, a reflux condenser, a dropping funnel and a nitrogen catheter. 50 g of petroleum ether with a boiling range of 90-120°C was used as a solvent. Nitrogen was introduced for 5-10 minutes while maintaining a weak nitrogen flow during the reaction. The mixture was heated under stirring to 110°C. The molar ratio of maleic anhydride to methyl 12-hydroxystearate was about 1:1, and the reaction time was 2.5 hours. 12-maleic acid monoester group-containing methyl stearate reaction intermediate was prepared, and the solvent was distilled off to give about 205.7 g of the reaction intermediate.

205.7 g of the reaction intermediate was charged into a reactor with a three-neck flask. 10 g of sodium tungstate was added as a catalyst, wherein the mass of sodium tungstate accounted for about 10% of that of the reaction intermediate. The mixture was heated to 65°C, and under stirring, 85 g of hydrogen peroxide solution (mass fraction: 30%, Shanghai Aladdin Biochemical Technology Co., Ltd.) was slowly dropwise added with the molar ratio of 12-maleic acid monoester group-containing methyl stearate to hydrogen peroxide of about 1:5. The dropwise adding rate was controlled to be 2 drops/second, and the reaction was conducted for 8 hours. The product was cooled, dried and filtered to give about 285.1 g of 12-epoxysuccinic acid monoester group-containing methyl stearate (3-(((18-methoxy-18-oxooctadec-7-yl)oxy)carbonyl)oxirane-2-carboxylic acid) product. The reaction formula is shown as Formula 3.

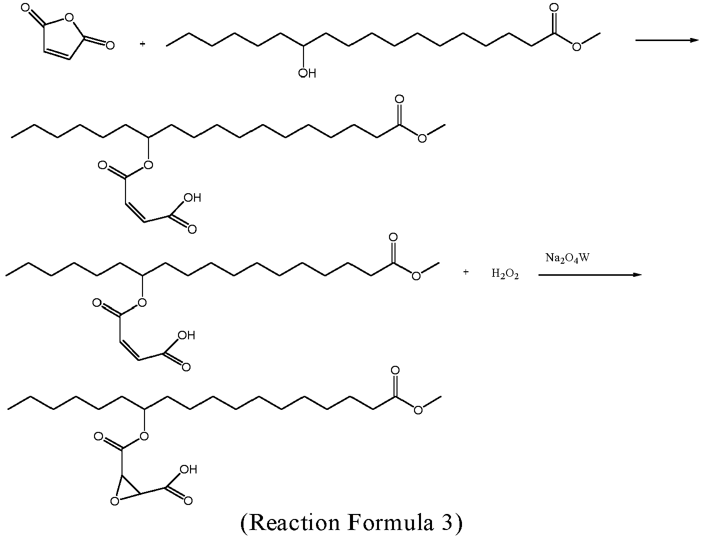

### Preparation Example 5

65 g (0.5 mol) glutaconic acid (97%, Beijing InnoChem Science & Technology Co., Ltd.) and 78 g (0.6 mol) iso-octanol (99%, Beijing InnoChem Science & Technology Co., Ltd.) were charged into a reactor equipped with an electric stirrer, a thermometer, a reflux condenser, a dropping funnel and a nitrogen catheter. 7.2 g of p-toluenesulfonic acid was added as a catalyst, wherein the amount of p-toluenesulfonic acid used was 5% of the total mass of the reactants. Nitrogen was introduced for 5-10 minutes while maintaining a weak nitrogen flow during the reaction. The mixture was heated under stirring to 160°C. The molar ratio of glutaconic acid to iso-octanol was about 1:1.2, and the reaction time was 5 hours. After cooling and standing to room temperature, mono-iso-octyl glutaconate reaction intermediate was prepared to give about 141.8 g of the reaction intermediate.

100 g of mono-iso-octyl glutaconate was charged into a reactor with a three-neck flask, and 10 g of sodium tungstate was added as a catalyst, wherein the mass of sodium tungstate accounted for about 10% of that of mono-iso-octyl glutaconate. 20 g of formic acid (analytical grade, Beijing InnoChem Science & Technology Co., Ltd.) was added as an oxygen carrier. The mixture was heated to 65°C, and under stirring, 94 g of hydrogen peroxide solution (mass fraction: 30%, Shanghai Aladdin Biochemical Technology Co., Ltd.) was slowly dropwise added with the molar ratio of mono-iso-octyl glutaconate to hydrogen peroxide of about 1:2. The dropwise adding rate was controlled to be 2 drops/second, and the reaction was conducted for 10 hours. The product was cooled, dried and filtered to give about 222.1 g of epoxidized mono-iso-octyl glutaconate product. The reaction formula is shown as Formula 4.

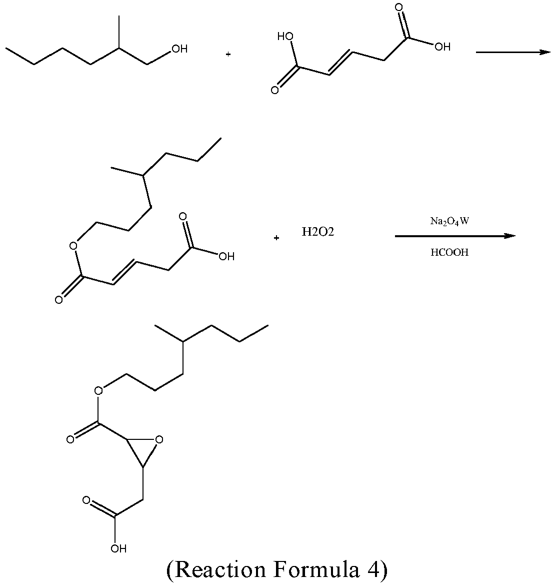

### Example 1

The lubricity modifier product prepared as shown in Preparation Example 1 was added to the blank diesel oil (having properties shown in Table 1) at 75 mg/kg. The lubricity of the additivated diesel oil was evaluated by a High-Frequency Reciprocating Rig (HFRR), and the results are shown in Table 2.

### Example 2

The lubricity modifier product prepared as shown in Preparation Example 1 was added to the blank diesel oil (having properties shown in Table 1) at 100 mg/kg. The lubricity of the additivated diesel oil was evaluated by a High-Frequency Reciprocating Rig (HFRR), and the results are shown in Table 2.

### Example 3

The lubricity modifier product prepared as shown in Preparation Example 1 was added to the blank diesel oil (having properties shown in Table 1) at 200 mg/kg. The lubricity of the additivated diesel oil was evaluated by a High-Frequency Reciprocating Rig (HFRR), and the results are shown in Table 2.

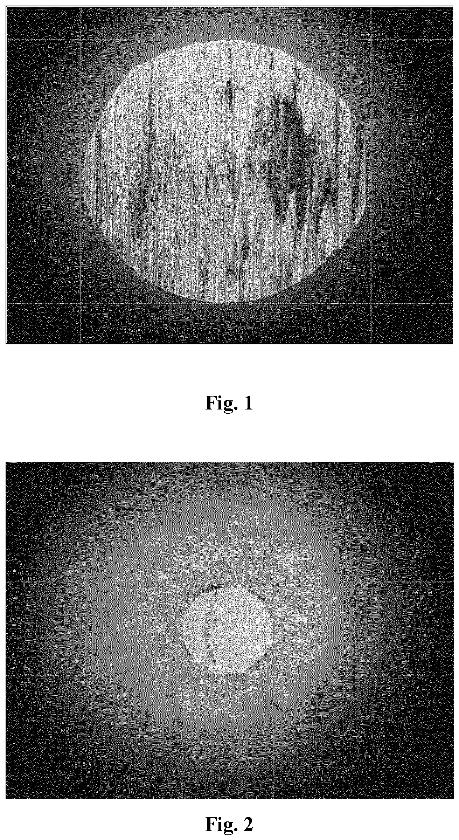

FIG. 1 as provided shows a HFRR wear scar graph of the blank diesel oil, wherein WS1.4 value is 640 µm. FIG. 2 shows a HFRR wear scar graph of the additivated diesel oil obtained by adding the lubricity modifier of the present application into the blank diesel oil in this Example, wherein WS1.4 value is 165 µm. As can be seen, the wear scar area of the additivated diesel oil is significantly smaller than that of the blank diesel oil, which indicates that the lubricity of the additivated diesel oil is significantly superior to that of the blank diesel oil.

### Example 4

The lubricity modifier product prepared as shown in Preparation Example 2 was added to the blank diesel oil (having properties shown in Table 1) at 75 mg/kg. The lubricity of the additivated diesel oil was evaluated by a High-Frequency Reciprocating Rig (HFRR), and the results are shown in Table 2.

### Example 5

The lubricity modifier product prepared as shown in Preparation Example 2 was added to the blank diesel oil (having properties shown in Table 1) at 100 mg/kg. The lubricity of the additivated diesel oil was evaluated by a High-Frequency Reciprocating Rig (HFRR), and the results are shown in Table 2.

### Example 6

The lubricity modifier product prepared as shown in Preparation Example 2 was added to the blank diesel oil (having properties shown in Table 1) at 200 mg/kg. The lubricity of the additivated diesel oil was evaluated by a High-Frequency Reciprocating Rig (HFRR), and the results are shown in Table 2.

### Example 7

The lubricity modifier product prepared in Preparation Example 3 was added to the blank diesel oil (having properties are shown in Table 1) at 100 mg/kg. The lubricity of the additivated diesel oil was evaluated by a High-Frequency Reciprocating Rig (HFRR), and the results are shown in Table 2.

### Example 8

The lubricity modifier product prepared in Preparation Example 4 was added to the blank diesel oil (having properties shown in Table 1) at 200 mg/kg. The lubricity of the additivated diesel oil was evaluated by a High-Frequency Reciprocating Rig (HFRR), and the results are shown in Table 2.

### Example 9

The lubricity modifier product prepared in Preparation Example 5 was added to the blank diesel oil (having properties shown in Table 1) at 75 mg/kg. The lubricity of the additivated diesel oil was evaluated by a High-Frequency Reciprocating Rig (HFRR), and the results are shown in Table 2.

### Example 10

The lubricity modifier product prepared in Preparation Example 5 was added to the blank diesel oil (having properties shown in Table 1) at 100 mg/kg. The lubricity of the additivated diesel oil was evaluated by a High-Frequency Reciprocating Rig (HFRR), and the results are shown in Table 2.

### Example 11

The lubricity modifier product prepared in Preparation Example 5 was added to the blank diesel oil (having properties shown in Table 1) at 200 mg/kg. The lubricity of the additivated diesel oil was evaluated by a High-Frequency Reciprocating Rig (HFRR), and the results are shown in Table 2.

### Comparative Example 1

49 g (0.5 mol) of maleic anhydride (analytical grade, Beijing InnoChem Science & Technology Co., Ltd.) and 143.5 g (1.1 mol) of iso-octanol (analytical grade, Beijing InnoChem Science & Technology Co., Ltd.) were charged into a reactor equipped with an electric stirrer, a thermometer, a reflux condenser, a dropping funnel and a nitrogen catheter. Nitrogen was introduced for 5-10 minutes while maintaining a weak nitrogen flow during the reaction. The mixture was heated under stirring to 120°C. The molar ratio of maleic anhydride to iso-octanol was about 1:2.2, and the reaction time was 4 hours. Di-iso-octyl maleate reaction intermediate was prepared to give about 190.6 g of the reaction intermediate.

190.6 g of the reaction intermediate was charged into a reactor with a three-neck flask, and 19 g of sodium tungstate was added as a catalyst, wherein the mass of sodium tungstate accounted for about 10% of that of the reaction intermediate. The mixture was heated to 65°C, and under stirring, 57.3 g of hydrogen peroxide solution (mass fraction: 30%, Shanghai Aladdin Biochemical Technology Co., Ltd.) was slowly dropwise added with the molar ratio of di-iso-octyl maleate to hydrogen peroxide of about 1:3. The dropwise adding rate was controlled to be 2 drops/second, and the reaction was conducted for 8 hours. The product was cooled, dried and filtered to give about 245.7 g of di-iso-octyl epoxysuccinate product.

The product was added to the blank diesel oil (having properties shown in Table 1) at a dosage of 200 mg/kg. The lubricity of the additivated diesel oil was evaluated, and the results are shown in Table 2.

### Comparative Example 2

49 g (0.5 mol) of maleic anhydride (analytical grade, Beijing InnoChem Science & Technology Co., Ltd.) and 78.5 g (0.6 mol) of iso-octanol (analytical grade, Beijing InnoChem Science & Technology Co., Ltd.) were charged into a reactor equipped with an electric stirrer, a thermometer, a reflux condenser, a dropping funnel and a nitrogen catheter. Nitrogen was introduced for 5-10 minutes while maintaining a weak nitrogen flow during the reaction. The mixture was heated under stirring to 105°C. The molar ratio of maleic anhydride to iso-octanol was about 1:1.2, and the reaction time was 2.5 hours. About 126.6 g of mono-iso-octyl maleate was prepared.

The mono-iso-octyl maleate was added to the blank diesel oil (having properties shown in Table 1) at a dosage of 200 mg/kg, 100 mg/kg and 75 mg/kg. The lubricity of the additivated diesel oil was evaluated and the results are shown in Table 2.

### Comparative Example 3

HiTEC^{®} Afton 4140 (commercial diesel lubricity modifier from Afton Chemical Corporation, USA) was added to the blank diesel oil (having properties shown in Table 1) at a dosage of 200 mg/kg.

### Comparative Example 4

Di-iso-octyl maleate (95%, purchased from Beijing InnoChem Science & Technology Co., Ltd.) was added to the blank diesel oil (having properties shown in Table 1) at a dosage of 200 mg/kg. The lubricity of the additivated diesel oil was evaluated and the results are shown in Table 2.

**Table 1. Physicochemical properties of blank diesel oil**

| Item | Result | Standard |
|---|---|---|
| Moisture (w)/% | None | GB/T 260 |
| Density (density at 20°C)/(kg/m³) | 821.7 | SH/T 0604 |
| Copper sheet corrosion (50°C, 3h)/rating | 1a | GB/T 5096 |
| Acidity/(mgKOH/100mL) | 0.44 | GB/T 258 |
| Initial boiling point /°C | 200.9 | GB/T 6536-C |
| 90% Recovery temperature/°C | 302.4 | |
| Final boiling point /°C | 343.4 | |
| Color/number | < 0.5 | GB/T 6540 |
| Cold filter plugging point/°C | -10 | SH/T 0248 |
| Mechanical impurities (w)/% | None | GB/T 511 |
| Light oil sulfur content /(mg/kg) | 6 | SH/T 0689 |
| Pour point /°C | -21 | GB/T 3535 |
| Freezing point /°C | -22 | GB/T 510 |
| Ash content (w)/% | <0.002 | GB/T 508 |
| Oxidation stability of diesel oil | <0.3 | SH/T 0175 |
| Closed cup flash point /°C | 79.6 | GB/T 261 |
| 10% carbon residue (w)/% | <0.05 | GB/T 17144-B |
| Viscosity (downstream method)/(mm²/s) | 2.6 | GB/T 265 |
| Lubricity, WS1.4, µm | 640 | SH/T 0765 |

Table 1 above shows the basic physicochemical properties of the blank diesel oil used, which is typically characterized by a lubricity (WS1.4) of 640 µm, not meeting the lubricity use requirements of diesel oils.

**Table 2. Lubricity evaluation results of diesel oils**

| Oil Sample | Dosage/mg·kg⁻¹ | WS 1.4/µm |
|---|---|---|
| Blank diesel oil | 0 | 640 |
| Example 1 | 75 | 376 |
| Example 2 | 100 | 209 |
| Example 3 | 200 | 165 |
| Example 4 | 75 | 395 |
| Example 5 | 100 | 224 |
| Example 6 | 200 | 218 |
| Example 7 | 100 | 354 |
| Example 8 | 200 | 204 |
| Example 9 | 75 | 422 |
| Example 10 | 100 | 337 |
| Example 11 | 200 | 172 |
| Comparative Example 1 | 200 | 644 |
| Comparative Example 2 | 75 | 552 |
| Comparative Example 2 | 100 | 375 |
| Comparative Example 2 | 200 | 237 |
| Comparative Example 3 | 200 | 424 |
| Comparative Example 4 | 200 | 637 |

It can be seen from Table 2 that the lubricity modifier product of the present application can significantly improve the lubricity of low-sulfur diesel oil when added in a small amount to the blank diesel oil. In addition, by comparing Examples 1-3 with Comparative Example 2, it can be seen that mono-iso-octyl epoxysuccinate has a lubricity improving effect superior to that of mono-iso-octyl maleate. Specifically, the lubricity improving effect of mono-iso-octyl epoxysuccinate is better than that of mono-iso-octyl maleate at the same dosage. Especially, at the dosage of 75 mg/kg, the lubricity improving effect of mono-iso-octyl maleate is relatively poor, while mono-iso-octyl epoxysuccinate can still maintain good lubricity improving effect. This finding is unexpected.

By comparing Examples 1-3 with Comparative Example 1, it can be seen that the lubricity improving effect of epoxidized monoester compound is definitely better than that of epoxidized diester compound. That is, on the premise of the same dosage, epoxidized monoester compound can significantly improve the lubricity of diesel oil, while epoxidized diester compound does not exhibit any lubricity improving effect.

On the other hand, the prepared lubricity modifier product is superior to the currently commercially available lubricity modifier products. On the premise of maintaining similar lubricity improving effect, the dosage of the lubricity modifier product provided by the present application in diesel oil is less than that of the commercially available lubricity modifier product.

### Example 12

The lubricity modifier product prepared as shown in Preparation Example 1 was added to 10W/40 lubricating oil by 2% (mass fraction). The anti-wear and anti-friction properties of the lubricating oil were evaluated by a four-ball friction and wear tester under test conditions of 75°C, 40 kg, and 1200 rpm, and the results are shown in Table 3.

### Example 13

The lubricity modifier product prepared as shown in Preparation Example 5 was added to 10W/40 lubricating oil by 2% (mass fraction). The anti-wear and anti-friction properties of the lubricating oil were evaluated by a four-ball friction and wear tester under test conditions of 75°C, 40 kg and 1200 rpm, and the results are shown in Table 3.

**Table 3. Lubricity evaluation results of lubricating oils**

| Oil Sample | Wear Scar Diameter/mm | Friction Coefficient |
|---|---|---|
| Commercially available oil | 0.4 | 0.107 |
| Example 10 | 0.36 | 0.103 |
| Example 11 | 0.33 | 0.101 |

As can be seen from Table 3, when the lubricity modifier product provided by the present application is added into the lubricating oil, the wear scar diameter and the friction coefficient are both reduced, and the anti-friction performance of the lubricating oil can be improved.

The lubricity of the additivated diesel oils obtained in Example 3, Comparative Example 1, Comparative Example 3 and Comparative Example 4 was evaluated by a High-Frequency Reciprocating Rig (HFRR) after storing the additivated diesel oils for a period of time under ambient conditions, and the results are shown in Table 4 below.

**Table 4. Stability evaluation of lubricity modifiers**

| Oil sample | Storage Period | Lubricity (WS 1.4)/µm |
|---|---|---|
| Example 3 | 0 day | 165 |
| Example 3 | 15 days | 209 |
| Example 3 | 30 days | 206 |
| Example 3 | 90 days | 172 |
| Example 3 | 120 days | 196.5 |
| Comparative Example 1 | 0 day | 644 |
| Comparative Example 1 | 60 days | 657 |
| Comparative Example 1 | 120 days | 659 |
| Comparative Example 3 | 0 day | 424 |
| Comparative Example 3 | 15 days | 410 |
| Comparative Example 3 | 30 days | 426 |
| Comparative Example 3 | 60 days | 432 |
| Comparative Example 3 | 90 days | 438 |
| Comparative Example 3 | 120 days | 441 |
| Comparative Example 4 | 0 day | 637 |
| Comparative Example 4 | 60 days | 664 |
| Comparative Example 4 | 120 days | 671 |

As can be seen from Table 4, the lubricity modifier products provided by the present application are able to maintain good lubricity improving performance over a long period of time under storage conditions. Specifically, the mono-iso-octyl epoxysuccinate lubricity modifier of the present application exhibits no significant change in lubricity improving effect upon storage for 15 days compared to 120 days at a dosage of 200 mg/kg, and still maintains good lubricity improving performance. In contrast, the lubricity modifier product of Comparative Example 3 exhibits a tendency of deterioration in lubricity improving effect at the same dosage as the storage time is prolonged. In addition, di-iso-octyl maleate used in Comparative Example 4 exhibits no lubricity improving effect on ultra-low-sulfur diesel oil with low lubricity, but exhibits a phenomenon of aggravating wear as the storage time is prolonged; and di-iso-octyl epoxysuccinate used in Comparative Example 1 exhibits no significant change in the performance as the storage time is prolonged, which indicates that the stability of the product is improved after the epoxidation reaction.

The present application is illustrated in detail hereinabove with reference to preferred embodiments, but is not intended to be limited to those embodiments. Various simple modifications may be made following the inventive concept of the present application, and these simple modifications shall be within the scope of the present application.

It should be noted that the various technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

## Claims

1. A lubricity modifier suitable for improving lubricity of oil products, comprising 50-100 wt%, preferably 60-100 wt%, more preferably 70-100 wt%, further preferably 80-100 wt%, particularly preferably 90-100 wt% of a compound having a structure represented by following formula (I), 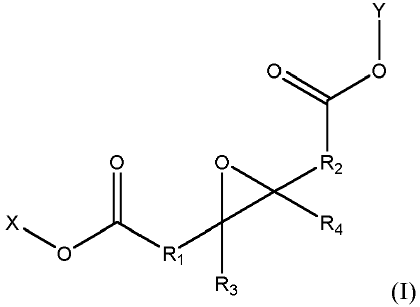
wherein, groups R₁ and R₂ are each independently a single bond or a substituted or unsubstituted C₁-C₂₀ hydrocarbylene group, preferably each independently a single bond or a substituted or unsubstituted C₁-C₁₀ alkylene group;
groups R₃ and R₄ are each independently hydrogen or a substituted or unsubstituted C₁-C₁₀ hydrocarbyl group, preferably each independently hydrogen or a substituted or unsubstituted C₁-C₅ hydrocarbyl group;
groups X and Y are each independently hydrogen or a substituted or unsubstituted C₁-C₅₀ hydrocarbyl group, preferably each independently hydrogen or a substituted or unsubstituted C₄-C₃₀ hydrocarbyl group, and at least one of X and Y is hydrogen, wherein the "substituted" refers to being substituted with at least one group selected from the group consisting of hydroxyl, carboxyl, ester group, hydrocarbyloxy, primary amino, secondary amino, amido, and aminoacyl, preferably selected from the group consisting of hydroxyl, carboxyl, ester group, and hydrocarbyloxy.

2. The lubricity modifier according to claim 1, wherein in the compound having the structure of formula (I), groups R₁ and R₂ are each independently a single bond or a substituted or unsubstituted C₁-C₄ alkylene group;
groups R₃ and R₄ are each independently hydrogen or a substituted or unsubstituted C₁-C₂ hydrocarbyl group;
groups X and Y are each independently hydrogen or a substituted or unsubstituted C₄-C₂₀ hydrocarbyl group, and at least one of X and Y is hydrogen, preferably one of X and Y is hydrogen and the other is a substituted or unsubstituted C₄-C₂₀ hydrocarbyl group;
wherein the "substituted" refers to being substituted with at least one group selected from the group consisting of hydroxyl, carboxyl, ester group and hydrocarbyloxy.

3. The lubricity modifier according to claim 1, wherein the compound having the structure of formula (I) is selected from the group consisting of epoxysuccinic acid monoester and epoxidized glutaconic acid monoester,
preferably, the compound having the structure of formula (I) is selected from the group consisting of mono-n-butyl ester, mono-iso-butyl ester, mono-n-pentyl ester, mono-iso-pentyl ester, mono-n-octyl ester, mono-iso-octyl ester, mono-n-nonyl ester, mono-iso-nonyl ester, mono-lauryl ester, mono-octadecyl ester, mono-oleyl ester, mono-cyclopentyl ester, mono-cyclohexyl ester, mono-poly(ethylene glycol) monomethyl ether ester, mono-poly(ethylene glycol) ester, mono-poly(propylene glycol) ester, mono-poly(propylene glycol) monobutyl ether ester of epoxysuccinic acid and epoxidized glutaconic acid, or any combination thereof, or selected from the group consisting of epoxysuccinic acid monoester group-containing ricinoleic acid, epoxysuccinic acid monoester group-containing methyl ricinoleate, 12-epoxysuccinic acid monoester group-containing stearic acid, 12-epoxysuccinic acid monoester group-containing methyl stearate, epoxidized glutaconic acid monoester group-containing ricinoleic acid, epoxidized glutaconic acid monoester group-containing methyl ricinoleate, 12-epoxidized glutaconic acid monoester group-containing stearic acid, 12-epoxidized glutaconic acid monoester group-containing methyl stearate, or any combination thereof.

4. A lubricating oil composition, comprising a base oil for lubricating oil and, based on mass of the base oil for lubricating oil, 0.01-30 wt%, preferably 0.05-20 wt%, further preferably 0.1-10 wt% of the compound having the structure of formula (I) as defined in any one of claims 1-3, wherein the compound is used as a lubricity modifier; preferably, the base oil for lubricating oil is selected from the group consisting of mineral base oils, synthetic base oils, and bio-base oils.

5. An engine fuel oil composition, comprising a liquid base fuel and, based on mass of the base fuel, 10-5000 mg/kg, preferably 50-1000 mg/kg, further preferably 100-500 mg/kg of the compound having the structure of formula (I) as defined in any one of claims 1-3, wherein the compound is used as a lubricity modifier,
preferably, the base fuel is selected from the group consisting of petroleum-based source fuels, such as middle distillate fuels, diesel fuels, gasoline, jet fuels, kerosene, marine oils, and other low-sulfur fuels, high asphaltene fuels, liquefied petroleum gas fuels, and the like; bio-renewable fuels, such as biodiesel fuels, biomass-to-liquid (BTL) fuels, and the like; synthetic fuels, such as gas-to-liquid (GTL) fuels and other synthetic fuels, and the like; coal-based source fuels, such as alcohols, ethers, Fischer-Tropsch synthetic fuels, coal-to-liquid (CTL) fuels, fuels derived from coal and/or petroleum coke; and other fuels, such as genetically engineered biofuels, and crops and extract fuels thereof; further preferably, the base fuel is selected from the group consisting of gasoline fuels and diesel fuels.

6. A method of using the compound having the structure of formula (I) as defined in any one of claims 1-3 as a lubricity modifier for oil products, comprising a step of adding the compound to an oil product,
preferably, the oil product is a lubricating oil or an engine fuel oil.

7. A method for improving lubricity of a lubricating oil, comprising adding to the lubricating oil 0.01-30 wt%, preferably 0.05-20 wt%, further preferably 0.1-10 wt%, based on mass of a base oil for lubricating oil in the lubricating oil, of the compound having the structure of formula (I) as defined in any one of claims 1-3 as a lubricity modifier.

8. A method for improving lubricity of an engine fuel oil, comprising adding to the engine fuel oil 10-5000 mg/kg, preferably 50-1000 mg/kg, further preferably 100-500 mg/kg, relative to mass of a base fuel in the engine fuel oil, of the compound having the structure of formula (I) as defined in any one of claims 1-3 as a lubricity modifier.

9. An additive composition for oil products, comprising from 10 wt% to less than 100 wt%, preferably 30-90 wt%, more preferably 50-90 wt%, of the compound having the structure of formula (I) as defined in any one of claims 1-3, and from greater than 0 wt% to 90 wt%, preferably 10-70 wt%, more preferably 10-50 wt%, of one or more additive for improving properties of oil products, wherein the compound having the structure of formula (I) is used as a lubricity modifier.

10. The additive composition for oil products according to claim 9, wherein the additive for improving properties of oil products is selected from the group consisting of detergent dispersants, metal deactivators, corrosion inhibitors, antioxidants, rust inhibitors, cetane improvers, pour point depressants, combustion promoters, or combinations thereof.
